# EUROPÄISCHE PATENTSCHRIFT

(11) **EP 0 891 427 B1**
(45) Veröffentlichungstag und Bekanntmachung des Hinweises auf die Patenterteilung: **18.06.2003**
(21) Anmeldenummer: 97918097.3
(22) Anmeldetag: 02.04.1997
(51) Int. Cl.: C12N 15/51, C12N 15/40, C12N 5/10, G01N 33/569, C07K 14/18

(54) **EXPRESSION VON HGV-ANTIGENEN UND DEREN VERWENDUNG**
EXPRESSION OF HGV ANTIGENS AND THEIR USE
EXPRESSION D'ANTIGENES DU VIRUS DE L'HEPATITE G ET LEUR UTILISATION

(30) Priorität: 03.04.1996 DE 19613406
(43) Veröffentlichungstag der Anmeldung: 20.01.1999
(73) Patentinhaber: Roche Diagnostics GmbH, 68305 Mannheim (DE)
(72) Erfinder: TACKE, Michael, D-82377 Penzberg (DE); ENGEL, Alfred, D-82377 Penzberg (DE)
(74) Vertreter: Weiss, Wolfgang, Dipl.-Chem. Dr.
(86) Internationale Anmeldenummer: EP9701649
(87) Internationale Veröffentlichungsnummer: WO97038108

(56) Entgegenhaltungen:
- EP-A- 0 747 482
- WO-A-95/21922
- WO-A-95/32291
- SCIENCE, Bd. 271, 26.Januar 1996, Seiten 505-508, XP002028989 LINNEN J ET AL: "MOLECULAR CLONING AND DISEASE ASSOCIATION OF HEPATITIS G VIRUS: A TRANSFUSION-TRANSMISSIBLE AGENT"
- LANCET THE, Bd. 349, 1.Februar 1997, LONDON GB, Seiten 318-320, XP002038169 TACKE M ET AL: "DETECTION OF ANTIBODIES TO A PUTATIVE HEPATITIS G VIRUS ENVELOPE PROTEIN"

## Beschreibung

Die vorliegende Erfindung betrifft neue Verfahren zur Herstellung von HGV-Antigenen und deren Verwendung zum diagnostischen Nachweis von HGV.

Neben Hepatitis-A-Virus (HAV) und Hepatitis-B-Virus (HBV), die schon seit langem bekannt sind, wurden in jüngerer Zeit weitere Viren charaktierisiert, die zwar mit Hepatitis assoziiert sind, jedoch eingenständige Gruppen von Viren darstellen. Diese Virengruppen werden üblicherweise so bezeichnet, daß einer neuen Gruppe immer ein fortlaufender Buchstabe des Alphabets zugeordnet wird. Jeder neu gefundene Hepatitis-assoziierte Virus wird auch gegenüber bereits bekannten Virengruppen abgegrenzt, daß er als nicht zur Gruppe der bisher bekannten Viren gehörend bezeichnet wird. So wurde Hepatitis-C-Virus (HCV) auch als NonA/NonB-Hepatitis-Virus bezeichnet (Choo et al. 1989, Science 244, 359 - 362). Die vorliegende Erfindug bezieht sich auf ein Virus, der nicht in die Gruppe der HAV-, HBV-, HCV-, HDV- und HEV-Viren eingeordnet werden kann und somit als Hepatitis-G-Virus (HGV) bezeichnet wird.

In WO 94/18217 ist ein Hepatitis-assoziiertes Virus beschrieben, das keiner der Gruppen HAV, HBV, HCV, HDV und HEV zugeordnet werden kann. Dieses Virus weist jedoch in seiner Nucleotidsequenz keinerlei Ähnlichkeit mit der in der vorliegenden Erfindung beschriebenen Sequenz auf.

In WO 95/21922 ist die Nucleinsäure- und Aminosäuresequenz von HGV beschrieben. In den Beispielen 13, 19 und 20 wird die rekombinante Expression von HGV-Polypeptiden in E.coli offenbart. Auf die in dieser Anmeldung offenbarten Sequenzen wird ausdrücklich Bezug genommen.

In WO 95/32291 ist ebenfalls die Nucleinsäure- und Aminosäuresequenz von HGV beschrieben. In Beispiel 16 wird die rekombinante Expression von HGV in E.coli, in Insektenzellen und in Vaccinia beschrieben. Auf die in dieser Anmeldung offenbarten Sequenzen wird ausdrücklich Bezug genommen.

Die in der vorliegenden Erfindung als HGV bezeichnete Gruppe von Hepatitis-assoziierten Viren zählt vermutlich zur Familie Flaviviridae (Chambers et al. 1990, Annu. Ref. Microbiol. 44, 649 - 688).

Virale Infektionen werden üblicherweise durch das Vorhandensein von viralen Antigenen oder/und Antikörpern gegen diese Antigene in Körperflüssigkeiten wie etwa Serum nachgewiesen. Zum Nachweis der Antikörper können virale Polypeptide oder Peptidfragmente davon als Antigene in einem Immunoassay eingesetz werden. Für die Durchführung eines solchen immunologischen Tests ist es erforderlich, geeignete immunogene Peptidoder Polypeptidbestandteile von Viren in ausreichender Menge bereitzustellen.

Bis heute sind keine Methoden zur routinemäßigen immunologischen Diagnose von HGV bekannt. Darüberhinaus ist bekannt, daß kurze synthetische Peptide sowie bisher bekannte rekombinante Polypeptide aus E.coli sich als diagnostische Reagenzien als nicht sonderlich gut erwiesen haben.

Die der vorliegenden Erfindung zurgrundeliegende Aufgabe war es demgemäß, neue Verfahren zur Expression von HGV-Antigenen bereitzustellen und neue Verfahren zur Detektion einer HGV-Infektion zu entwickeln.

Diese Aufgabe wird gelöst, durch ein neues Verfahren zur rekombinanten Herstellung von Antigenen aus HGV in membrangebundener oder/und löslicher Form. Als HGV-Antigen im Sinne der vorliegenden Erfindung sind Polypeptide aus dem HGV-Genom (WO 95/21922 und WO 95/32291) geeignet, die mindestens eine antigene oder/und immunogene Determinante aufweisen. Aus dem HGV-Gesamtgenom sind die DNA-Sequenzbereiche bevorzugt, die für die putativen Hüllproteine E1 und E2 kodieren. Diese Hüllproteine sind zusammengesetzt aus einem aminoterminalen Hauptabschnitt, der an der Außenseite eines funktionellen Viruspartikels lokalisiert ist und eine entscheidende Rolle beim Andockken an den Wirtsorganismus spielt, und einem kurzen carboxyterminalen hydrophoben Abschnitt, der in der Membran verankert ist.

Das Ziel der Erfindung war es, ein möglichst authentisches Expressionssystem für HGV-Antigene bereitzustellen, welches eine korrekte posttranslationale Prozessierung, gegebenenfalls eine Glycosilierung und den Export aus dem Cytoplasma gewährleistet. Hierzu wird die Expression insbesondere in Säugerzellen, z.B. in CHO-Zellen durchgeführt. Dort findet die Proteinbiosynthese an den Ribosomen im Cytosol statt. Bei Expression der HGV-Antigene in operativer Verknüpfung mit aminoterminalen Signalsequenzen, z.B. 20 bis 30 Aminosäuren lange hydrophobe Sequenzen, die während der Proteinbiosynthese im Cytosol von sogenannten Signalerkennungsteilchen (signal recognition particles) erkannt werden, werden die Ribosomen zum endoplasmatischen Reticulum (ER) dirigiert. Dort werden die entstehenden Polypeptidketten durch die ER-Membran geschleust, bis sie durch Stop-Transfersequenzen in der Membran arretiert werden. Im Lumen des ER werden die Proteine gegebenenfalls glycosiliert und anschließend im Golgi-Apparat weiter modifiziert. Schließlich werden sie für den Export in Richtung Plasmamembran sortiert.

Gemäß einer ersten Ausführungsform betrifft die vorliegende Erfindung ein Verfahren zur rekombinanten Herstellung eines Antigens von HGV in membrangebundener Form, umfassend die Schritte:
(a) Einführen einer DNA-Sequenz in eine Wirtszelle, wobei die DNA-Sequenz für das Antigen mit einer funktionell aktiven Membrankerdomäne in operativer Verknüpfung mit einem Signalpeptid kodiert, wobei das Signalpeptid einen Export des Antigens aus dem Cytoplasma bewirkt,
(b) Kultivieren der Wirszelle aus Schritt (a) unter Bedingungen, bei denen eine Expression der eingeführten DNA-Sequenz erfolgt, wobei das Antigen in membrangebundener Form erzeugt wird, und
(c) Gewinnen der das Antigen in seiner Membran enhaltenden Wirtszelle.

Vorzugsweise werden die Hüllproteine E1 oder/und E2 als membrangebundene Antigene hergestellt. Die Nucleotidsequenz eines E1-Antigens von HGV ist beispielsweise die zwischen Position 127 und Position 681 von SEQ ID NO. 1 angegebene Nucleotidsequenz, die für ein Polypeptid mit der zwischen Position 39 und 223 von SEQ ID NO. 2 angegebenen Aminosäuresequenz kodiert. Eine Nucleotidsequenz, die für ein E2-Antigen von HGV kodiert, ist beispielsweise die zwischen Position 127 und 1290 von SEQ ID NO. 5 angegebene Nucleotidsequenz. Diese Nucleotidsequenz kodiert für ein Polypeptid mit der zwischen Position 39 und 426 von SEQ ID NO. 6 angegebenen Aminosäuresequenz. Neben diesen spezifisch angegebenen Nucleotid- bzw. Aminosäuresequenzen umfaßt die vorliegende Erfindung jedoch auch Varianten dieser Sequenzen.

Gegenstand der vorliegenden Erfindung ist somit ein Verfahren, bei dem man ein membrangebundenes Antigen E1 herstellt, welches kodiert ist von
(a) der zwischen Position 127 und 681 von SEQ ID NO. 1 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequen.

Vorzugsweise umfaßt das membrangebundene Antigen E1
(a) die zwischen Position 39 und 223 von SEQ ID NO. 2 angegebene Aminosäuresequenz oder
(b) eine mit der Sequenz aus (a) mindestens 80 % homologe und insbesondere mindestens 90 % homologe Aminosäuresequenz.

Ein weiterer Gegenstand der vorliegenden Erfindung ist ein Verfahren zur Herstellung eines membrangebundenen Antigens E2, welches kodiert ist von
(a) der zwischen Position 127 und 1290 von SEQ ID NO. 5 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequenz.

Vorzugsweise umfaßt das membrangebundene Antigen E2
(a) die zwischen Position 39 und 426 von SEQ ID NO. 6 angegebene Aminosäuresequenz oder
(b) eine mit der Sequenz aus (a) mindestens 80 % homologe und insbesondere mindestens 90 % homologe Aminosäuresequenz.

Der Begriff "Hybridisierung" gemäß vorliegender Erfindung wird wie bei Sambrook et al. (1989) in Molecular Cloning: A Laboratory Manual, Cold Spring Harbor Laboratory, New York, 1.101 - 1.104 verwendet. Demzufolge spricht man von einer Hybridisierung, wenn nach Waschen für eine Stunde mit 1 X SSC und 0,1 % SDS bei 55°C, vorzugsweise bei 62°C und besonders bevorzugt bei 68°C, insbesondere für 1 Stunde in 0,2 X SSC und 0,1 % SDS bei 55°C, vorzugsweise bei 62°C und besonders bevorzugt bei 68°C noch ein positives Hybridisierungssignal beobachtet wird.
Ein weiterer Gegenstand der vorliegenden Erfindung ist ein rekombinante Zelle, insbesondere eine in vitro kultivierte Säugerzelle wie etwa eine CHO-Zelle, die an ihrer Oberfläche HGV-Antigene in membrangebundener Form präsentiert, insbesondere die Antigene E1 oder/und E2 oder immunologisch relevante Teilsequenzen davon.

Die erfindungsgemäße Zelle kann als diagnostisches Reagenz zum Nachweis von HGV, z.B. durch FACS-Analyse oder durch ELISA verwendet werden. Hierzu wird die Reaktion einer Zelle, die ein HGV-Antigen an ihrer Oberfläche präsentiert, mit einer Probeflüssigkeit, z.B. Humanserum, bestimmt. Bei Auftreten einer Reaktion ist davon auszugehen, daß das in der getesteten Probe Anti-HGV-Antikörper enthalten sind.

Der zweite Aspekt der vorliegenden Erfindung betrifft ein Verfahren zur rekombinanten Herstellung eines HGV-Antigens, vorzugsweise des E1 oder des E2-Antigens in löslicher Form. Dieses Verfahren umfaßt die Schritte:
(a) Einführen einer DNA-Sequenz in eine Wirtszelle, wobei die DNA-Sequenz für das Antigen mit einer funktionell inaktiven Membranankerdomäne kodiert;
(b) Kultivieren der Wirtszelle aus Schritt (a) unter Bedingungen, bei denen eine Expression der eingeführten DNA-Sequenz erfolgt, wobei das Antigen in löslicher Form erzeugt wird, und
(c) Gewinnen des Antigens.

Vorzugsweise wird das Verfahren so durchgeführt, daß die für das Antigen kodierende DNA-Sequenz operativ mit einer Signalsequenz verknüpft ist, die einen Export des Antigens aus dem Cytoplasma der Wirtszelle bewirkt. In diesem Fall ist eine einfache Gewinnung des Antigens aus dem Kulturüberstand ohne weitere Reinigung möglich. Vorzugsweise wird eine eukaryontische Wirtszelle, z.B. eine CHO-Zelle verwendet.

Beispiele für HGV-Antigene in löslicher Form, die durch das erfindungsgemäße Verfahren erhältlich sind, sind die Antigene E1 und E2 mit einer durch Deletion funktionell inaktivierten Ankermembrandomäne. Ein lösliches Antigen E1 wird vorzugsweise kodiert von
(a) der zwischen Position 127 und 552 von SEQ ID NO. 3 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequenz.

Besonders bevorzugt umfaßt das lösliche Antigen E1
(a) die zwischen Position 39 und 180 von SEQ ID NO. 4 angegebene Aminosäuresequenz oder
(b) eine mit der Sequenz aus (a) mindestens 80 % und insbesondere mindestens 90 % homologe Aminosäuresequenz.

Ein bevorzugtes lösliches Antigen E2 ist kodiert von
(a) der zwischen Position 127 und 1134 von SEQ ID NO. 7 angegebenen Nucleotidsequenz, oder
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequenz.

Besonders bevorzugt umfaßt ein lösliches Antigen E2
(a) die zwischen Position 39 und 374 von SEQ ID NO. 8 angegebene Aminosäuresequenz oder
(b) eine mit der Sequenz aus (a) mindestens 80 % und insbesondere mindestens 90 % homologe Aminosäuresequenz.

Ein weiterer Gegenstand der vorliegenden Erfindung ist eine Nucleinsäure, die für das Antigen E1 oder E2 von HGV mit einer funktionell inaktiven Membranankerdomäne kodiert. Vorzugsweise umfaßt diese Nucleinsäure eine wie zuvor definierte Sequenz.

Noch ein weiterer Gegenstand der vorliegenden Erfindung ist ein von der Nucleinsäure kodiertes Polypeptid, das zur Anwendung in einem diagnostischen Test vorzugsweise mindestens eine Markierungsgruppe oder eine Festphasenbindegruppe trägt. Als Markierungsgruppe kommen alle bekannten Markierungsgruppen in Frage, die in einem Testsystem nachgewiesen werden können, d.h. direkt oder indirekt nachweisbare Markierungsgruppen. Dabei ist unter einer direkt nachweisbaren Markierungsgruppe eine solche Gruppe zu verstehen, die ein direkt nachweisbares Signal erzeugt, z.B. eine radioaktive Gruppe, eine Enzymgruppe oder eine Lumineszenzgruppe. Besonders bevorzugt sind Enzymgruppen und Lumineszenzgruppen, insbesondere Elektrochemilumineszenzgruppen. Andererseits kann die Markierungsgruppe auch eine indirekt nachweisbare Gruppe sein, z.B. eine Biotin- oder Hapten-Gruppe, die durch Reaktion mit einem geeigneten Bindepartner (Streptavidin, Avidin bzw. Anti-Hapten-Antikörper), der wiederum eine signalerzeugende Gruppe trägt, nachweisbar ist. Als Markierungsgruppe kann ebenfalls eine an das erfindungsgemäße HGV-Polypeptid anfusionierte heterologe Peptidoder Polypeptiddomäne verwendet werden, die mit einem geeigneten Bindepartner spezifisch reagiert. Ein Beispiel hierfür ist das FLAG-Epitop, das aufgrund seiner Reaktion mit einem Antikörper als Markierungsgruppe dienen kann.

Peptid-, Polypeptid-, Hapten- oder Biotin-Gruppen können auch als Festphasenbindegruppen zur Immobilisierung der Antigene an einer Festphase eingesetzt werden. Die Markierungs- bzw. Festphasenbindegruppe kann, sofern erforderlich, mit dem Antigen auf bekannte Weise gekoppelt werden, beispielsweise über einen bifunktionellen Spacer. Derartige Verfahren zur Kopplung von Markierungs- oder Festphasenbindegruppen an Polypeptide sind einem Fachmann auf dem Gebiet der Immunologie bekannt und brauchen hier nicht näher erläutert werden.

Noch ein weiterer Gegenstand der vorliegenden Erfindung ist ein rekombinanter Vektor, der mindestens eine Kopie einer für ein lösliches HGV-Antigen kodierenden Nucleinsäure enthält. Dieser Vektor ist vorzugsweise ein eukaryontischer Vektor, z.B. ein Hefevektor oder ein für höhere Zellen geeigneter Vektor, z.B. ein Plasmidvektor oder viraler Vektor. Derartige Vektoren sind dem Fachmann bekannt (vgl. z.B. Sambrook et al., Supra, Kapitel 16).

Ein weiterer Gegenstand der vorliegenden Erfindung ist eine Zelle, die mit einer erfindungsgemäßen DNA-Sequenz oder einem erfindungsgemäßen Vektor transformiert ist. Diese Zelle ist vorzugsweise eine eukaryontische Zelle, insbesondere eine Säugerzelle. Verfahren zur Transformation bzw. Transfektion eukaryontischer Zellen mit exogenen Nucleinsäuresequenzen sind dem Fachmann auf dem Gebiet der Molekularbiologie geläufig und brauchen hier nicht näher erläutert zu werden (vgl. z.B. Sambrook et al., Kapitel 16).

Das lösliche HGV-Polypeptid kann als Diagnostikreagenz zum Nachweis von HGV und aufgrund seiner antigenen Wirkung auch zur Herstellung eines Impfstoffs gegen eine HGV-Infektion verwendet werden.

Sowohl lösliche als auch membranbeständige HGV-Antigene können als solche oder als Fusionen mit anderen Peptid- oder/und Polypeptidkomponenten erzeugt werden, z.B. als Fusion mit einer immunologisch reaktiven Peptidkomponente wie etwa dem FLAG-Epitop.

Der Nachweis von HGV erfolgt insbesondere durch immunologische Bestimmung eines gegen HGV gerichteten Antikörpers in einer Probeflüssigkeit, wobei man die Probeflüssigkeit mit mindestens einem erfindungsgemäßen löslichen HGV-Polypeptid inkubiert und die Antikörper über eine Bindung mit dem Polypeptid nachweist. Dieses immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. Vorzugsweise wird ein heterogenes Testformat verwendet, bei dem das Vorhandensein des Antikörpers in Anwesenheit einer Festphase nachgewiesen wird.

Eine Ausführungsform dieses Testformats ist das sogenannte Doppelantigen-Brückentest-Konzept. Dabei wird die Probeflüssigkeit mit mindestens zwei HGV-Polypeptiden P1 und P2 inkubiert, wobei das Polypeptid P1 an eine Festphase gebunden ist oder in einer an eine Festphase-bindefähigen Form vorliegt und das Polypeptid P2 eine Markierungsgruppe trägt. Der Antikörper in der Probeflüssigkeit wird durch Bestimmung in der Markierung in der Festphase oder/und in der flüssigen Phase, vorzugsweise in der Festphase, über einen immobilisierten, d.h. an die Festphase gebundenen Immunkomplex nachgewiesen.

Die Testdurchführung beinhaltet vorzugsweise ein Mischen der Probeflüssigkeit mit einem markierten Antigen P2 sowie mit einem festphasenseitigen Antigen P1, um einen markierten immobilisierten Komplex aus markiertem Antigen, Antikörper und Festphasengebundenem Antigen zu erhalten. Durch Verwendung der erfindungsgemäßen Polypeptide in löslicher Form gelingt es, Leerwerterhöhungen und eine ungünstige Signal/Rausch-Relation aufgrund von Agregationen des Antigens zu vermeiden.

Das erfindungsgemäße lösliche HGV-Polypeptid kann aber auch in anderen Testformaten eingesetzt werden, beispielsweise in einem indirekten Immunoassay zur Erkennung von spezifischem Immunglobulin durch Bindung an ein wandseitiges spezifisches Antigen und indirekten Nachweis über ein Konjugat mit einem zweiten Anti-HGV-Antikörper, der vorzugsweise eine Markierung trägt.

Schließlich betrifft die vorliegende Erfindung auch ein Reagenz zur immunologischen Bestimmung eines gegen Heptatitis-G-Virus gerichteten Antikörpers, der neben anderen Nachweiskomponenten eine rekombinante Zelle mit einem membrangebundenen HGV-Antigen oder/und ein lösliches HGV-Antigen enthält.

Weiterhin wird die vorliegende Erfindung durch die nachfolgenden Beispiele, Sequenzprotokolle und Abbildungen beschrieben.
Es zeigen:
- SEQ ID NO. 1:: eine Nucleotidsequenz, die für ein das E1-Antigen von HGV einschließlich Transmembrandomäne enthaltendes Fusionsprotein (HGV-E1 TM) kodiert,
- SEQ ID NO. 2:: die Aminosäuresequenz des Fusionsproteins,
- SEQ ID NO. 3:: eine Nucleotidsequenz, die für ein das E1-Antigen von HGV ohne Transmembrandomäne kodierendes Fusionsprotein (HGV-E1S) kodiert,
- SEQ ID NO. 4:: die Aminosäuresequenz des Fusionsproteins HGV-E1S,
- SEQ ID NO. 5:: eine Nucleotidsequenz, die für ein das E2-Antigen von HGV mit Transmembrandomäne (HGV-E2TM) enthaltendes Fustionsprotein kodiert,
- SEQ ID NO. 6:: die Aminosäuresequenz des Fusionsproteins HGV-E2TM,
- SEQ ID NO. 7:: eine Nucleotidsequenz, die eine das E2-Antigen von HGV ohne die Transmembrandomäne enthaltendes Fusionsprotein kodiert,
- SEQ ID NO. 8:: die Aminosäuresequenz des Fusionsproteins HGV-E2S und

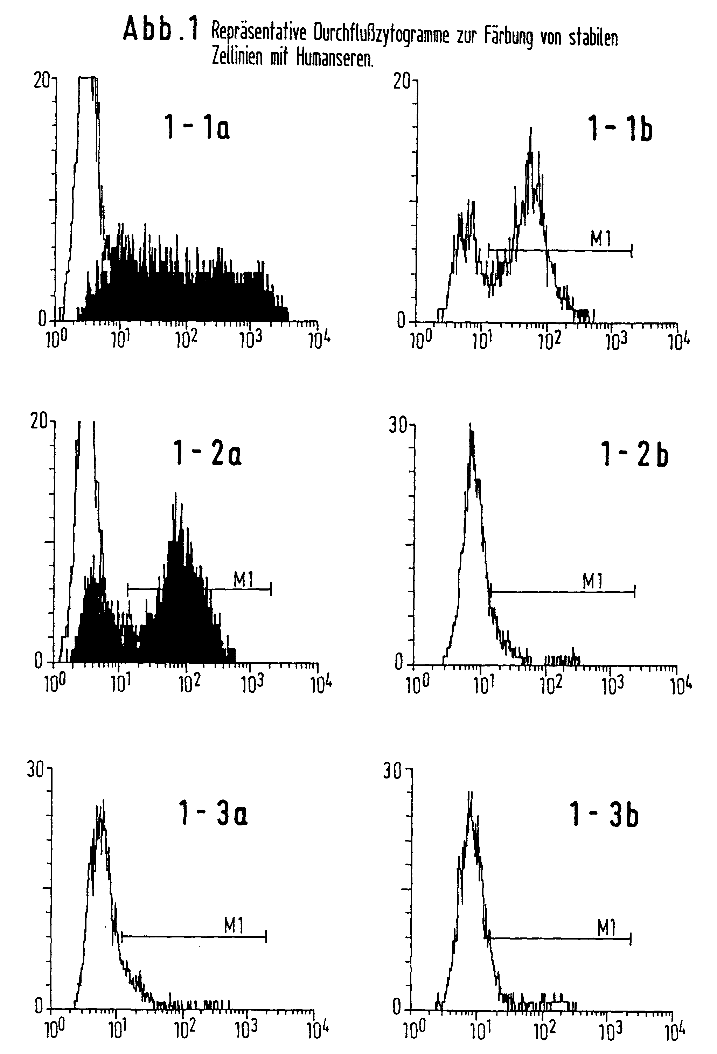

- Abb. 1:: repräsentative Durchflußcytogramme, welche die Anfärbung von E2-Antigen exprimierenden Zellinien mit Humanseren zeigen.

### BEISPIELE

### Beispiel 1:

### Klonierung und Expression von HGV-Antigenen

Zur Manipulation von DNA und zur Expression von Polypeptiden wurden Standardmethoden benutzt, wie sie bei Sambrook et al. (1989) in Molecular cloning: A Laboratory Manual, Cold Spring Harbor Laboratory, New York und Ausubel et al. (1989) in Current Protocols Molecular Biology, John Wiley & Sons, New York beschrieben sind.

Die Manipulation von DNA wurde im E.coli K12-Stamm DH5α durchgeführt. Die Expression der rekombinanten Proteine erfolgte in CHO-DHFR-Zellen, DSM ACC 126.
1.1. Als Vektor zur Expression in eukaryontischen Zellsystem wurde ein Derivat des Vektors pcDNA3 mit CMV-Promotor und BGH poly-Adenylierungssignal (Invitrogen BV, NV Leek, Niederlande) verwendet. Hierzu wurde der Vektor pcDNA3 durch den Austausch des Neomycin-Resistenzgens gegen ein Dihydrofolatreduktase (DHFR) Gen modifiziert. Dies erfolgte durch Restriktionsspaltung von pcDNA3 mit Avr-II/Bst1107I, Isolierung des ca. 4 kB langen Vektorfragments und Insertion eines ca. 700 Bp langen AvrII/bst1107I DHFR-Fragments (Setzer et al. (1982), J. Biol. Chem. 257, 5143 - 5147; Crouse et al. (1982), J. Biol. Chem. 257, 7887 - 7897).
   Die für die HGV-Hüllproteine E1 und E2 kodierenden DNA-Sequenzen wurden in den resultierenden Vektor pcDNA3-DHFR in operativer Verknüpfung an eine Signalpeptidsequenz sowie an eine für ein sogenanntes FLAG-Epitop kodierende DNA-Sequenz kloniert und zusammen mit beiden Elementen als Fusionsprotein exprimiert. Als Signalsequenz wurde die Erythropoietinsignalsequenz (Jacobs et al. (1985) Nature 313, 806 - 810) als 93 Bp langes EcoRI/EheI-Fragment (Position 1 bis 93 von SEQ ID NO. 1) eingesetzt.
   Das FLAG-Epitop ist ein 8 Aminosäuren langes Peptid (Hopp et al. (1988), Bio/Technology 6, 1204 - 1210), gegen das ein monoklonaler Antikörper kommerziell erhältlich ist, welcher gegebenenfalls zur Aufreinigung und Identifizierung des gewünschten Expressionsprodukts eingesetzt werden kann. Zur Herstellung der für das FLAG-Epitop kodierenden DNA-Sequenz wurden zwei Oligonucleotide mit einander hybridisiert und nach Kinasierung als Linker eingesetzt (entsprechend Position 94 - 125 von SEQ ID NO. 1).
   Zur Herstellung des E1- bzw. E2 Expressionsvektors wurde das Plasmid pcDNA3-DHFR mit EcoRI und NotI verdaut, das ca. 5,9 kB lange Vektorfragment isoliert und in einer 3-Wege-Ligation mit dem Signalsequenzfragment und dem FLAG-Linker ligiert.
   Die verwendete HGV-cDNA stammte von Genelabs Technologies Inc., Redwood City, CA, USA (WO 95/32291). Das fast vollständige HGV-Genom lag als ca. 9,3 kB langes XbaI/EcoRI-Fragment vor, welches in die entsprechenden Restriktionsstellen des Vektors pGEM-3Z (Promega Corp., Madison, WI, USA) kloniert worden war.
   Es wurde eine Expression der Hüllproteine E1 und E2 mit bzw. ohne Transmembranankersequenz durchgeführt. Die Nucleotid- bzw. Aminosäuresequenzen für die jeweiligen Konstrukte sind wie folgt angegeben:
   SEQ ID NO. 1/2-Antigen E1 mit Transmembransequenz (Position 13 - 93 Signalpeptid; Position 94 - 126 FLAG-Sequenz + NotI-Linker, Position 127 - 681 E1 Sequenz, Position 688 - 693 XbaI Linker).
   SEQ ID NO.3/4-E1 Antigen ohne Transmembransequenz (Position 13 - 93 Signalsequenz, Position 94 - 126 FLAG-Sequenz + Linker, Position 127 - 552 E1 Sequenz, Position 559 - 564 Linkersequenz).
   SEQ ID NO.5/6-E2 Antigen mit Transmembransequenz (Position 13 - 93 EPO-Sequenz, Position 94 - 126 FLAG-Sequenz + Linker, Position 127 - 1290 E2 Sequenz, Position 1297 - 1302 Linkersequenz)
   SEQ ID NO.7/8-E2 Antigen ohne Transmembranankersequenz (Position 13 - 93 Signalsequenz, Position 94 - 126 FLAG-Sequenz + Linker, Position 127 - 1134 E2 Sequenz, Position 1141 - 1146 Linkersequenz).
   Die Klonierung der obigen Konstrukte erfolgte durch Amplifizierung der für die Hüllproteine kodierenden DNA-Sequenzen mit geeigneten Oligonucleotiden mittels PCR und Klonierung als NotI/XbaI-Fragmente in frame an die für das Signalpeptid und das FLAG-Epitop kodierenden DNA-Sequenzen in den Vektor.
1.2. Expression
   Zur Expression der HGV-Hüllproteine wurden die unter 1.1. beschriebenen Expressionskonstrukte unter Verwendung des Transfektionsreagenz DOTAP (Boehringer Mannheim GmbH, Kat.Nr. 1202 375) in CHO-DHFR-Zellen eingeschleust. Die Selektion transfizierter Zellen erfolgte in Nucleosidfreiem MEM α-Medium (GibcoBRL, Kat.Nr. 22561-021) versetzt mit 10 % dialysiertem, fötalem Kälberserum (GibcoBRL, Kat.Nr. 10110-070).
   Durch DHFR-Selektion wurde das Expressionskonstrukt in stabilen Zellinien weiter durch Zugabe von Methotrexat (Sigma-Aldrich GmbH, Kat.Nr. M8407) im Wachstumsmedium amplifiziert. Nach 10tägiger Selektion wurden einzelne Klone gepickt und mittels Immunfluoreszenz unter Verwendung von Anti-FLAG M1 Antikörper (Eastman Kodak Comp., Kat.Nr. IB13001) auf Expression des FLAG-Epitops analysiert.
   Alternativ hierzu wurden Klone, welche E1- und E2 Sequenzen mit Transmembrananker auf der Zellmembran präsentieren sollten (HGV-E1TM und HGV-E2TM) nach Färbung mit M1-Antikörper und Anti-Maus Ig-Fluorescein-(Fab')₂ Fragment (Boehringer Mannheim GmbH, Kat.Nr. 1214 616) mittels Durchflußzytometrie analysiert (siehe Beispiel 2). Die das FLAG-Epitop exprimierenden Klone wurden subkloniert und für die Analyse von Humanseren verwendet (siehe FACS-Analytik, Beispiel 2).
   Klone, welche die Expressionskonstrukte HGV-E1s und HGV-E2s exprimieren, präsentieren die Antigene nicht membranständig, sondern sezernieren diese ohne Transmembrananker direkt in den Kulturüberstand. Diese Antigene können in klassischen immunologischen Tests eingesetzt werden. Dazu ist nicht einmal eine Reinigung erforderlich, vielmehr können sie direkt aus dem serumfreien Kulturüberstand an mit M1-Antikörper beschichtete ELISA-Platten gekoppelt werden.

### Beispiel 2:

### FACS-Analytik mit dem membrangebundenen Antigen HGV-E2TM

Bei diesem Verfahren wurden CHO-Zellen, die ein rekombinantes FLAG-E2TM-Fusionsprotein membranständig exprimieren, als Fängersubstanz für HGV-spezifische Antikörper in Humanseren verwendet. Dabei stand das rekombinante exprimierte FLAG-E2TM-Fusionsprotein in nativer Form als Fänger für Antikörper zur Verfügung. Auf diese Weise blieben Konformations-abhängige Epitope erhalten, so daß diese Methode ideal ist, um menschliche Seren auf die Anwesenheit von HGV-spezifischen Antikörpern hin zu untersuchen.

Die FLAG-E2TM exprimierenden Zellen wurden hierzu von dem Kulturgefäß in 0,04 % EDTA in PBS abgelöst und in PBS gewaschen. Je 200.000 Zellen wurden in 100 µl PBS, 1 mM CaCl₂, 0,2 % Rinderserumalbumin und 0,02 % NaN₃ resuspendiert, mit 1 - 5 µl Humanserum für 30 Min auf Eis inkubiert, mit dem gleichen Puffer zweimal gewaschen und für weitere 30 Min mit Anti-Human Ig-Fluorescein (Fab')₂ Fragment (Boehringer Mannheim GmbH, Kat.Nr. 1295750) inkubiert, um die an das HGV gebundenen Antikörper für die Durchflußzytometrie nachweisbar zu machen. Nach wiederum zweimaligem Waschen wurden die Zellen im Durchflußzytometer analysiert.

Um unspezifische Bindung von Serumantikörpern an CHO-Zellen oder eine Bindung nur an das FLAG-Epitop auszuschließen, kann man zusätzlich CHO-Zellen, die mit einem irrelevanten FLAG-Fusionsprotein transfiziert wurden (z.B. dem humanen Urokinase Rezeptor, der ebenfalls mit einer aminoterminalen FLAG-Sequenz versehen ist) als Negativ-Referenz färben.

Abbildung 1-1 zeigt die Färbung zweier CHO-Klone, die den Urokinase-Rezeptor mit aminoterminaler FLAG-Sequenz (3UR8, Abb. 1-1a) bzw. E2TM mit aminoterminaler FLAG-Sequenz (2E2TM12, Abb. 1-1b) exprimieren. Beide Klone wurden mit dem FLAG-spezifischen Antikörper M1 angefärbt (ausgefüllte Kurve). Die nicht ausgefüllten Kurven repräsentieren die Isotyp-Kontrolle. Man sieht ein große Expressionsbreite von FLAG bei 3UR8 bzw. im Fall 2E2TM12, d.h., daß ca. 2/3 der Zellen des Klons das rekombinante Fusionsprotein exprimieren.

Abb. 1-2 zeigt die Färbung mit einem Humanserum, welches HGV-spezifische Antikörper enthält. 2/3 der 2E2TM12-Zellen wurden mit diesem Serum angefärbt (Abb. 1-2b), analog zur Anfärbung mit dem M1-Antikörper (Abb. 1-1b). Dagegen wurde keine Anfärbung von 3UR8-Zellen gefunden (Abb. 1-2a), obwohl sie das FLAG-Epitop exprimieren. Die Färbung der 2E2TM12-Zellen kann also nicht auf einer Bindung von Serumantikörpern an CHO-Zellen oder das FLAG-Epitop beruhen, sondern nur auf einer spezifischen Bindung an den E2TM-Anteil des rekombinanten Fusionsproteins. Das Verfahren ist somit geeignet, E2TM-spezifische Antikörper in Humanseren nachzuweisen.

Abb. 1-3 zeigt schließlich die Anfärbung mit einem Humanserum, das keine HGV-spezifischen Antikörper enthält. Weder 2E2TM12-Zellen (Abb. 1-3a) noch 3UR8-Zellen (Abb. 1-3b) wurden mit diesem Serum angefärbt. Dies zeigt, daß die membrangebundene Expression von HGV-Hüllproteinen ein spezifisches diagnostisches Instrument zum Nachweis von HGV-Antikörpern in Humanserum ist.

### Beispiel 3:

### ELISA-Analytik mit dem Antigen HGV-E2TM

Bei diesem Verfahren wurde das FLAG-E2TM-Fusionsprotein aus den CHO-Klonen isoliert und als Fängersubstanz für HGV-spezifische Antikörper im klassischen ELISA-Format verwendet. Das Antigen wurde dabei über das FLAG-Epitop an einen biotinylierten M1-Antikörper gebunden, welcher seinerseits an eine Streptavidin-beschichtete ELISA-Platte gekoppelt war. Auch in dieser Form wurde das rekombinant exprimierte FLAG-E2TM-Fusionsprotein in nativer Form präsentiert, Konformations-abhängige Epitope blieben erhalten.
a) Vorbereitung der ELISA-Platten: Streptavidin-beschichtete ELISA-Mikrotierplatten (Microcode, Streptavidin MTP F8) wurden 60 min mit 25 µl FLAG-spezifischen biotinylierten M1 Antikörper (2,5 µg/ml in PBS, 0,2 % Rinderserumalbumin) inkubiert und anschließend dreimal mit 0,9 % NaCl, 0,05 % Tween 20 gewaschen. Anschließend wurde 30 min mit PBS, 0,2 % Rinderserumalbumin, 0,05 % Tween 20 blockiert und erneut wie oben gewaschen.
b) Zellyse: Die FLAG-E2TM exprimierenden Zellen wurden von dem Kulturgefäß in 0,04 % EDTA in PBS abgelöst und in PBS gewaschen. Anschließend wurden die Zellen in PBS, 0,5 % Nonidet P40, Protease-Mix (Boehringer Mann GmbH, Kat.Nr. 1206 893) lysiert. Dazu wurden je 10⁷ Zellen/ml Solubilisierungslösung für zwei Stunden auf Eis inkubiert. Ungelöstes Material wurde durch Zentrifugation abgetrennt.
c) Kopplung der Antigene und Assay: Mit dem Zellysat (verdünnt in PBS, 0,1 % Nonidet P40) wurden die präparierten ELISA-Platten (siehe a) für eine Stunde bei Raumtemperatur inkubiert. Nach dreimaligem Waschen mit PBS, 0,1 % Nonidet P40 wurde schließlich das Humanserum in geeigneter Verdünnung (1 : 30 - 1 : 50) zugegeben und 90 min bei Raumteperatur inkubiert. Nach mehrmaligem Waschen zuerst mit PBS, 0,1 % Nonidet P40 und dann mit 0,9 % NaCl, 0,05 % Tween 20 wurde schließlich eine Stunde mit anti-Human Ig-POD, (Fab')₂ Fragment (Boehringer Mannheim GmbH, Kat.Nr. 1089 196) inkubiert. Nach intensivem Waschen mit 0,9 % NaCl, 0,05 % Tween 20 wurde ABTS® als Subtrat eingesetzt und die Farbänderung nach 30 bis 60 min im ELISA-Reader bei 405 nm gemessen.

### Ergebnis:

Zunächst wurden in diesem Assay als Nullproben CHO-Lysate eingesetzt, die kein rekombinantes E2TM exprimierten. Mit diesen wurden Extinktionswerte im Bereich von durchschnittlich 100 bis 140 mE in einem Blutspenderkollektiv beobachtet. Folg-lich wurden solche Proben als positiv bewertet, die eine Extinktion von mindestens 250 mE und ein Signalverhältnis E2/CHO von mindestens 2,0 aufwiesen. Auf diese Weise ergab sich eine sehr gute Überlappung mit den Resultaten aus der FACS-Analytik (Beispiel 2), die als sehr spezifisch einzustufen sind.

Innerhalb eines Blutspender-Panels waren 7 von 80 Proben (9 %) positiv im Hinblick auf die Anwesenheit von Antikörpern gegen das E2-Antigen.

Innerhalb eines Risikogruppen-Panels, bestehend aus 122 Drogenabhängigen (sog. "intravenus drug abusers") waren 40 Proben (33 %) E2-ELISA positiv.

Darüber hinaus wurde in einer Reihe von Verlaufsseren (entnommen von je einem Patienten mit Post-Transfusions-Hepatitis C, Post-Transfusions-Hepatitis NonBNonC, Post-Transfusions-Hepatits B sowie sporadischer, akuter Hepatitis B) Serokonversionen von HGV-E2 negativ zu HGV-E2 positiv beobachtet.

Vergleichbare Ergebnisse wurden auch in einem homogenen ELISA Verfahren erhalten. Dabei wurden die E2TM enthaltenden Lysate zusammen mit dem biotinylierten M1-Antikörper und Seren (in einer 1 : 50 Verdünnung) für 2 Stunden bei Raumtemperatur in den Streptavidin-beschichteten Mikrotiterplatten (Microcode, Streptavidin MTP F8) inkubiert und anschließend zuerst mit PBS, 0,1 % Nonidet P40 und dann mit 0,9 % NaCl, 0,05 % Tween 20 gewaschen, gefolgt von einer Stunde Inkubation mit anti-Human Ig-POD, (Fab')₂ Fragment (Boheringer Mannheim GmbH, Kat.Nr. 1089 196). Nach intensivem Waschen mit 0,9 % NaCl, 0,05 % Tween 20 wurde ABTS® als Substrat eingesetzt und die Farbänderung nach 30 bis 60 min im ELISA-Reader bei 405 gemessen.

## Patentansprüche

1. Verfahren zur rekombinanten Herstellung eines Antigens von HGV in membrangebundener Form, umfassend die Schritte:
(a) Einführen einer DNA-Sequenz in eine Wirtszelle, wobei die DNA-Sequenz für das Antigen mit einer funktionell aktiven Membranankerdomäne in operativer Verknüpfung mit einem Signalpeptid kodiert, wobei das Signalpeptid einen Export des Antigens aus dem Cytoplasma bewirkt,
(b) Kultivieren der Wirtszelle aus Schritt (a) unter Bedingungen, bei denen eine Expression der eingeführten DNA-Sequenz erfolgt, wobei das Antigen in membrangebundener Form erzeugt wird und
(c) Gewinnen der das Antigen in seiner Membran enthaltenden Wirtszelle.

2. Verfahren nach Anspruch 1,
**dadurch gekennzeichnet,**
**dass** man ein membrangebundenes Antigen E1 herstellt, welches kodiert ist von
(a) der zwischen Position 127 und 681 von SEQ ID NO. 1 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nukleotidsequenz, wobei eine Hybridisierung nach Waschen für 1 Stunde mit 1x SSC und 0,1 % SDS bei 55 °C erfolgt.

3. Verfahren nach einem der Ansprüche 1 bis 2,
**dadurch gekennzeichnet,**
**dass** man ein membrangebundenes Antigen E1 herstellt, umfassend
(a) die zwischen Position 39 und 223 von SEQ ID NO. 2 angegebene Aminosäuresequenz oder
(b) eine mit der Sequenz aus (a) mindestens 80 % homologe Aminosäuresequenz.

4. Verfahren nach einem der Ansprüche 1 bis 2,
**dadurch gekennzeichnet,**
**dass** man ein membrangebundenes Antigen E2 herstellt, welches kodiert ist von
(a) der zwischen Position 127 und 1290 von SEQ ID NO. 5 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nukleotidsequenz, wobei eine Hybridisierung nach Waschen für 1 Stunde mit 1x SSC und 0,1 % SDS bei 55 °C erfolgt.

5. Verfahren nach einem der Ansprüche 1 oder 4,
**dadurch gekennzeichnet,**
**dass** man ein membrangebundenes Antigen E2 herstellt, umfassend
(a) die zwischen Position 39 und 426 von SEQ ID NO. 6 angegebenen Aminosäuresequenz oder
(b) eine mit der Sequenz aus (a) mindestens 80 % homologe Aminosäuresequenz.

6. Rekombinante Zelle,
**dadurch gekennzeichnet,**
**dass** sie an ihrer Oberfläche die Antigene E1 oder/und E2 von HGV in membrangebundener Form präsentiert.

7. Verwendung einer Zelle nach Anspruch 6 als Diagnostikreagenz zum Nachweis von HGV.

8. Verfahren zur rekombinanten Herstellung des Antigens E1 von HGV in löslicher Form, umfassend die Schritte:
(a) Einführen einer DNA-Sequenz in eine Wirtszelle, wobei die DNA-Sequenz für das Antigen mit einer funktionell inaktiven Membranankerdomäne kodiert, wobei die DNA-Sequenz operativ mit einer Signalsequenz verknüpft ist, die einen Export des Antigens aus dem Cytoplasma der Wirtszelle bewirkt.
(b) Kultivieren der Wirtszelle aus Schritt (a) unter Bedingungen, bei denen eine Expression der eingeführten DNA-Sequenz erfolgt, wobei das Antigen in löslicher Form erzeugt wird, und
(c) Gewinnen des Antigens aus dem Kulturüberstand.

9. Verfahren nach Anspruch 8,
**dadurch gekennzeichnet,**
**dass** man ein lösliches Antigen E1 herstellt, welches kodiert ist von
(a) der zwischen Position 127 und 552 von SEQ ID NO. 3 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nukleotidsequenz, wobei eine Hybridisierung nach Waschen für 1 Stunde mit 1x SSC und 0,1 % SDS bei 55 °C erfolgt.

10. Verfahren nach Anspruch 8 oder 9,
**dadurch gekennzeichnet,**
**dass** man ein lösliches Antigen E1 herstellt, umfassend
(a) die zwischen Position 39 und 180 von SEQ ID NO. 4 angegebene Aminosäuresequenz oder
(b) eine mit der Sequenz aus (a) mindestens 80 % homologe Aminosäuresequenz.

11. Nucleinsäure,
**dadurch gekennzeichnet,**
**dass** sie für das Antigen E1 von HGV mit einer funktionell inaktiven Membranankerdomäne kodiert und operativ mit einer Signalsequenz verknüpft ist, die einen Export des Antigens aus dem Cytoplasma einer Wirtszelle bewirkt.

12. Nucleinsäure nach Anspruch 11,
**dadurch gekennzeichnet,**
**dass** sie
(a) die in SEQ ID NO. 3 von Position 127-552 angegebene Nucleotidsequenz,
(b) eine der Sequenz aus (a) im Rahmen der Degeneration des genetischen Codes entsprechende Nucleotidsäuresequenz oder/und
(c) eine mit der Sequenz aus (a) oder/und (b) unter stringenten Bedingungen hybridisierende Nucleotidsequenz umfasst, wobei eine Hybridisierung nach Waschen für 1 Stunde mit 1 x SSC und 0,1 % SDS bei 55 °C erfolgt.

13. Polypeptid,
**dadurch gekennzeichnet,**
**dass** es von einer Nucleinsäure nach Anspruch 11 oder 12 kodiert ist.

14. Rekombinanter Vektor,
**dadurch gekennzeichnet,**
**dass** er mindestens eine Kopie einer Nucleinsäure nach Anspruch 11 oder 12 enthält.

15. Rekombinante Zelle,
**dadurch gekennzeichnet,**
**dass** sie mit einer Nucleinsäure nach Anspruch 11 oder 12 oder einem Vektor nach Anspruch 14 transformiert ist.

16. Verwendung eines durch ein Verfahren nach einem der Ansprüche 8 bis 10 hergestellten Polypeptids oder eines Polypeptids nach Anspruch 13 als Diagnostikreagenz zum in vitro Nachweis von HGV.

17. Reagenzienkit zum Nachweis von HGV,
**dadurch gekennzeichnet,**
**dass** er neben anderen Nachweiskomponenten
(a) eine rekombinante Zelle nach Anspruch 7 oder/und
(b) ein durch ein Verfahren nach einem der Ansprüche 8 bis 10 hergestelltes Polypeptid oder ein Polypeptid nach Anspruch 13 enthält.

## Claims

1. Process for the recombinant production of an antigen of HGV in a membrane-bound form comprising the steps:
(a) introducing a DNA sequence into a host cell in which the DNA sequence codes for the antigen with a functionally active membrane anchor domain in operative linkage with a signal peptide and the signal peptide causes the antigen to be exported from the cytoplasm,
(b) culturing the host cell from step (a) under conditions in which the introduced DNA sequence is expressed whereby the antigen is produced in a membrane-bound form and
(c) isolating the host cell containing the antigen in its membrane.

2. Process as claimed in claim 1,
**wherein**
a membrane-bound antigen E1 is produced which is coded by
(a) the nucleotide sequence shown between position 127 and 681 of SEQ ID NO.1,
(b) a sequence corresponding to the sequence from (a) within the scope of the degeneracy of the genetic code or/and
(c) a nucleotide sequence hybridizing with the sequences from (a) or/and (b) under stringent conditions, the hybridization taking place after washing for 1 hour with 1 x SSC and 0.1 % SDS at 55°C.

3. Process as claimed in one of the claims 1 to 2,
**wherein**
a membrane-bound antigen E1 is produced comprising
(a) the amino acid sequence shown between position 39 and 223 of SEQ ID NO.2 or
(b) an amino acid sequence that is at least 80 % homologous to the sequence from (a).

4. Process as claimed in one of the claims 1 to 2,
**wherein**
a membrane-bound antigen E2 is produced which is coded by
(a) the nucleotide sequence shown between position 127 and 1290 of SEQ ID NO.5,
(b) a sequence corresponding to the sequence from (a) within the scope of the degeneracy of the genetic code or/and
(c) a nucleotide sequence hybridizing with the sequences from (a) or/and (b) under stringent conditions, the hybridization taking place after washing for 1 hour with 1 x SSC and 0.1 % SDS at 55°C.

5. Process as claimed in one of the claims 1 or 4,
**wherein**
a membrane-bound antigen E2 is produced which comprises
(a) the amino acid sequence shown between position 39 and 426 of SEQ ID NO.6 or
(b) an amino acid sequence that is at least 80 % homologous to the sequence from (a).

6. Recombinant cell,
**wherein**
it presents the antigens E1 or/and E2 of HGV in a membrane-bound form on its surface.

7. Use of a cell as claimed in claim 6 as a diagnostic reagent to detect HGV.

8. Process for the recombinant production of the antigen E1 of HGV in a soluble form comprising the steps:
(a) introducing a DNA sequence into a host cell, the DNA sequence coding for the antigen with a functionally inactive membrane anchor domain and being operatively linked with a signal sequence which causes the antigen to be exported from the cytoplasm of the host cell,
(b) culturing the host cell from step (a) under conditions in which the introduced DNA sequence is expressed whereby the antigen is produced in a soluble form and
(c) isolating the antigen from the culture supernatant.

9. Process as claimed in claim 8,
**wherein**
a soluble antigen E1 is produced which is coded by
(a) the nucleotide sequence shown between position 127 and 552 of SEQ ID NO.3,
(b) a sequence corresponding to the sequence from (a) within the scope of the degeneracy of the genetic code or/and
(c) a nucleotide sequence hybridizing with the sequences from (a) or/and (b) under stringent conditions, the hybridization taking place after washing for 1 hour with 1 x SSC and 0.1 % SDS at 55°C.

10. Process as claimed in claim 8 or 9,
**wherein**
a soluble antigen E1 is produced comprising
(a) the amino acid sequence shown between position 39 and 180 of SEQ ID NO.4 or
(b) an amino acid sequence that is at least 80 % homologous to the sequence from (a).

11. Nucleic acid,
**wherein**
it codes for the antigen E1 of HGV with a functionally inactive membrane anchor domain and is operatively linked with a signal sequence which results in the export of the antigen from the cytoplasm of a host cell.

12. Nucleic acid as claimed in claim 11,
**wherein**
it comprises
(a) the nucleotide sequence shown in SEQ ID NO. 3 from position 127 - 552,
(b) a nucleotide sequence corresponding to the sequence from (a) within the scope of the degeneracy of the genetic code or/and
(c) a nucleotide sequence hybridizing with the sequences from (a) or/and (b) under stringent conditions, the hybridization taking place after washing for 1 hour with 1 x SSC and 0.1 % SDS at 55°C.

13. Polypeptide,
**wherein**
it is coded by a nucleic acid as claimed in claim 11 or 12.

14. Recombinant vector,
**wherein**
it contains at least one copy of a nucleic acid as claimed in claim 11 or 12.

15. Recombinant cell,
**wherein**
it is transformed by a nucleic acid as claimed in claim 11 or 12 or a vector as claimed in claim 14.

16. Use of a polypeptide produced by a process according to one of the claims 8 to 10 or of a polypeptide as claimed in claim 13 as a diagnostic reagent for the in vitro detection of HGV.

17. Reagent kit for the detection of HGV,
**wherein**
it contains in addition to other detection components
(a) a recombinant cell as claimed in claim 7 or/and
(b) a polypeptide produced by a process as claimed in one of the claims 8 to 10 or a polypeptide as claimed in claim 13.

## Revendications

1. Procédé de fabrication recombinante d'un antigène du VHG sous forme liée à la membrane, comprenant les étapes suivantes :
(a) introduire une séquence d'ADN dans une cellule hôte, la séquence d'ADN codant l'antigène avec un domaine d'ancrage à la membrane fonctionnellement actif en relation fonctionnelle avec un peptide signal, le peptide signal entraînant l'exportation de l'antigène hors du cytoplasme,
(b) cultiver la cellule hôte de l'étape (a) dans des conditions dans lesquelles une expression de la séquence d'ADN introduite a lieu, l'antigène étant produit sous une forme liée à la membrane et
(c) récolter la cellule hôte contenant l'antigène dans sa membrane.

2. Procédé selon la revendication 1, **caractérisé en ce qu'**on produit un antigène E1 lié à la membrane qui est codé par
(a) la séquence nucléotidique donnée entre les positions 127 et 681 de SEQ ID N°1,
(b) une séquence correspondant à la séquence de (a) en tenant compte de la dégénérescence du code génétique ou/et
(c) une séquence de nucléotides s'hybridant avec les séquences de (a) ou/et (b) dans des conditions stringentes, une hybridation ayant lieu après lavage pendant 1 heure avec 1 x SSC et 0,1% SDS à 55°C.

3. Procédé selon l'une des revendications 1 à 2, **caractérisé en ce que** l'on produit un antigène E1 lié à la membrane comprenant
(a) la séquence d'acides aminés donnée entre les positions 39 et 223 de SEQ ID N°2 ou
(b) une séquence d'acides aminés homologue à au moins 80% à la séquence de (a).

4. Procédé selon l'une des revendications 1 à 2, **caractérisé en ce que** l'on produit un antigène E2 lié à la membrane qui est codé par
(a) la séquence de nucléotides donnée entre les positions 127 et 1290 de SEQ ID N°5,
(b) une séquence correspondant à la séquence de (a) en tenant compte la dégénérescence du code génétique ou/et
(c) une séquence de nucléotides s'hybridant avec la séquence de (a) ou/et de (b) dans des conditions stringentes, une hybridation ayant lieu après lavage pendant 1 heure avec 1 x SSC et 0,1% SDS à 55°C.

5. Procédé selon l'une des revendications 1 ou 4, **caractérisée en ce que** l'on produit un antigène E2 lié à la membrane comprenant
(a) la séquence d'acides aminés donnée entre les positions 39 et 426 de SEC ID N°6 ou
(b) une séquence d'acides aminés homologue à au moins 80% à la séquence de (a).

6. Cellule recombinante **caractérisée en ce qu'**elle présente à sa surface l'antigène E1 ou/et E2 du VHG sous forme liée à la membrane.

7. Utilisation d'une cellule selon la revendication 6 comme réactif de diagnostic pour le dépistage du VHG.

8. Procédé de fabrication recombinante de l'antigène E1 du VHG sous forme soluble, comprenant les étapes suivantes :
(a) introduire une séquence d'ADN dans une cellule hôte, la séquence d'ADN codant l'antigène avec un domaine d'ancrage membranaire fonctionnellement inactif, la séquence d'ADN étant liée de manière fonctionnelle avec une séquence signal qui entraîne l'exportation de l'antigène hors du cytoplasme de la cellule hôte.
(b) cultiver la cellule hôte de l'étape (a) dans des conditions dans lesquelles une expression de la séquence d'ADN introduite a lieu, l'antigène étant produit sous forme soluble et
(c) récolter l'antigène dans le surnageant de culture.

9. Procédé selon la revendication 8 **caractérisé en ce que** l'on produit un antigène E1 soluble qui est codé par
(a) la séquence de nucléotides donnée entre les positions 127 et 552 de SEQ ID N°3,
(b) une séquence correspondant à la séquence de (a) en tenant compte de la dégénérescence du code génétique ou/et
(c) une séquence de nucléotides s'hybridant avec les séquences de (a) ou/et (b) dans des conditions stringentes, l'hybridation ayant lieu après lavage pendant 1 heure avec 1 x SSC et 0,1% SDS à 55°C.

10. Procédé selon les revendications 8 ou 9 **caractérisé en ce que** l'on produit un antigène E1 soluble comprenant
(a) la séquence d'acides aminés donnée entre les positions 39 et 180 de SEQ ID N°4 ou
(b) une séquence d'acides aminés homologue à au moins 80% avec la séquence de (a).

11. Acide nucléique **caractérisé en ce qu'**il code l'antigène E1 du VHG avec un domaine d'ancrage à la membrane fonctionnellement inactif et en-ce qu'il est lié de manière fonctionnelle à une séquence signal qui entraîne une exportation de l'antigène hors du cytoplasme d'une cellule hôte.

12. Acide nucléique selon la revendication 11 **caractérisé en ce qu'**il contient
(a) la séquence de nucléotides donnée en position 127-552 dans SEQ ID N°3,
(b) une des séquences d'acide nucléotidique correspondant à la séquence de (a) en tenant compte de la dégénérescence du code génétique ou/et
(c) une séquence de nucléotides s'hybridant avec la séquence de (a) ou/et (b) dans des conditions stringentes, une hybridation ayant lieu après lavage pendant une heure avec 1 x SSC et 0,1% SDS à 55°C.

13. Polypeptide **caractérisé en ce qu'**il est codé par un acide nucléique selon la revendication 11 ou 12.

14. Vecteur recombinant **caractérisé en ce qu'**il contient au moins une copie d'un acide nucléique selon la revendication 11 ou 12.

15. Cellule recombinante **caractérisée en ce qu'**elle est transformée avec un acide nucléique selon la revendication 11 ou 12 ou avec un vecteur selon la revendication 14.

16. Utilisation d'un polypeptide produit par un procédé selon l'une des revendications 8 à 10 ou d'un polypeptide selon la revendication 13 comme réactif de diagnostic pour le dépistage *in vitro* du VHG.

17. Trousse de réactif pour le dépistage du VHG **caractérisée en ce qu'**elle contient, outre d'autres composants de dépistage
(a) une cellule recombinante selon la revendication 7 ou/et
(b) un polypeptide produit par un procédé selon l'une des revendications 8 à 10 ou un polypeptide selon la revendication 13.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 112-132
      (C) ORT: Mannheim
      (E) LAND: DE
      (F) POSTLEITZAHL: 68305
   (ii) BEZEICHNUNG DER ERFINDUNG: Expression von HGV-Antigenen und deren Verwendung
   (iii) ANZAHL DER SEQUENZEN: 8
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 693 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Hepatitis G Virus
   (vii) UNMITTELBARE HERKUNFT:
      (B) CLON(E) : E1TM
   (ix) MERKMAL:
      (A) NAME/SCHLÜSSEL: CDS
      (B) LAGE:13..681
   (xi) SEQUENZBESCHREIBUNG: SEQ ID NO: 1:
   GAATTCACCA CC ATG GGG GTG CAC GAA TGT CCT GCC TGG CTG TGG CTT           48
                 Met Gly Val His Glu Cys Pro Ala Trp Leu Trp Leu
                   1               5                   10
   CTC CTG TCC CTG CTG TCG CTC CCT CTG GGC CTC CCA GTC CTG GGC GAC         96
   Leu Leu Ser Leu Leu Ser Leu Pro Leu Gly Leu Pro Val Leu Gly Asp
           15                  20                  25
   TAC AAG GAC GAT GAC GAT AAG GCG GCC GCC ATT CTG GCC CCG GCC ACC        144
   Tyr Lys Asp Asp Asp Asp Lys Ala Ala Ala Ile Leu Ala Pro Ala Thr
       30                  35                  40
   CAC GCT TGT CGA GCG AAT GGG CAA TAT TTC CTC ACA AAT TGT TGT GCC        192
   His Ala Cys Arg Ala Asn Gly Gln Tyr Phe Leu Thr Asn Cys Cys Ala
   45                  50                  55                  60
   CCG GAG GAC ATC GGG TTC TGC CTG GAG GGT GGA TGC CTG GTG GCC CTG        240
   Pro Glu Asp Ile Gly Phe Cys Leu Glu Gly Gly Cys Leu Val Ala Leu
                   65                  70                  75
   GGG TGC ACG ATT TGC ACT GAC CAA TGC TGG CCA CTG TAT CAG GCG GGT        288
   Gly Cys Thr Ile Cys Thr Asp Gln Cys Trp Pro Leu Tyr Gln Ala Gly
               80                  85                  90
   TTG GCT GTG CGG CCT GGC AAG TCC GCG GCC CAA CTG GTG GGG GAG CTG        336
   Leu Ala Val Arg Pro Gly Lys Ser Ala Ala Gln Leu Val Gly Glu Leu
           95                  100                 105
   GGT AGC CTA TAC GGG CCC CTG TCG GTC TCG GCC TAT GTG GCT GGG ATC        384
   Gly Ser Leu Tyr Gly Pro Leu Ser Val Ser Ala Tyr Val Ala Gly Ile
       110                 115                 120
   CTG GGC CTG GGT GAG GTG TAC TCG GGT GTC CTA ACG GTG GGA GTC GCG        432
   Leu Gly Leu Gly Glu Val Tyr Ser Gly Val Leu Thr Val Gly Val Ala
   125                 130                 135                 140
   TTG ACG CGC CGG ATC TAC CCG GTG CCT AAC CTG ACG TGT GCA GTC GCG        480
   Leu Thr Arg Arg Ile Tyr Pro Val Pro Asn Leu Thr Cys Ala Val Ala
                   145                 150                 155
   TGT GAG TTA AAG TGG GAA AGT GAG TTT TGG AGA TGG ACT GAA CAG CTG        528
   Cys Glu Leu Lys Trp Glu Ser Glu Phe Trp Arg Trp Thr Glu Gln Leu
               160                 165                 170
   GCC TCC AAC TAC TGG ATT CTG GAA TAC CTC TGG AAG GTC CCA TTT GAT        576
   Ala Ser Asn Tyr Trp Ile Leu Glu Tyr Leu Trp Lys Val Pro Phe Asp
           175                 180                 185
   TTC TGG AGA GGC GTG ATA AGC CTG ACC CCC TTG TTG GTT TGC GTG GCC        624
   Phe Trp Arg Gly Val Ile Ser Leu Thr Pro Leu Leu Val Cys Val Ala
       190                 195                 200
   GCA TTG CTG CTG CTT GAG CAA CGG GTT GTC ATG GTC TTC CTG TTG GTG        672
   Ala Leu Leu Leu Leu Glu Gln Arg Val Val Met Val Phe Leu Leu Val
   205                 210                 215                 220
   ACG ATG GCC TAATAGTCTA GA                                              693
   Thr Met Ala
(2) ANGABEN ZU SEQ ID NO: 2:
   (i) SEQUENZKENNZEICHEN:
      (A) LÄNGE: 223 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Gly Val His Glu Cys Pro Ala Trp Leu Trp Leu Leu Leu Ser Leu
     1               5                  10                  15
   Leu Ser Leu Pro Leu Gly Leu Pro Val Leu Gly Asp Tyr Lys Asp Asp
               20                  25                  30
   Asp Asp Lys Ala Ala Ala Ile Leu Ala Pro Ala Thr His Ala Cys Arg
           35                  40                  45
   Ala Asn Gly Gln Tyr Phe Leu Thr Asn Cys Cys Ala Pro Glu Asp Ile
       50                  55                  60
   Gly Phe Cys Leu Glu Gly Gly Cys Leu Val Ala Leu Gly Cys Thr Ile
   65                  70                  75                  80
   Cys Thr Asp Gln Cys Trp Pro Leu Tyr Gln Ala Gly Leu Ala Val Arg
                   85                  90                  95
   Pro Gly Lys Ser Ala Ala Gln Leu Val Gly Glu Leu Gly Ser Leu Tyr
               100                 105                 110
   Gly Pro Leu Ser Val Ser Ala Tyr Val Ala Gly Ile Leu Gly Leu Gly
           115                 120                 125
   Glu Val Tyr Ser Gly Val Leu Thr Val Gly Val Ala Leu Thr Arg Arg
       130                 135                 140
   Ile Tyr Pro Val Pro Asn Leu Thr Cys Ala Val Ala Cys Glu Leu Lys
   145                 150                 155                 160
   Trp Glu Ser Glu Phe Trp Arg Trp Thr Glu Gln Leu Ala Ser Asn Tyr
                   165                 170                 175
   Trp Ile Leu Glu Tyr Leu Trp Lys Val Pro Phe Asp Phe Trp Arg Gly
               180                 185                 190
   Val Ile Ser Leu Thr Pro Leu Leu Val Cys Val Ala Ala Leu Leu Leu
           195                 200                 205
   Leu Glu Gln Arg Val Val Met Val Phe Leu Leu Val Thr Met Ala
       210                 215                 220
(2) ANGABEN ZU SEQ ID NO: 3:
   (i) SEQUENZKENNZEICHEN:
      (A) LÄNGE: 564 Basenpaare
      (B) ART: Nucleotid
      (C) STRANGFORM: beides
      (D) TOPOLOGIE: linear
   (vi) URSPRÜNLICHE HERKUNFT:
      (A) ORGANISMUS: Hepatitis G Virus
   (vii) UNMITTELBARE HERKUNFT:
      (B) CLON(E): E1S
   (ix) MERKMAL:
      (A) NAME/SCHLÜSSEL: CDS
      (B) LAGE:13..552
   (xi) SEQUENZBESCHREIBUNG: SEQ ID NO: 3:
   GAATTCACCA CC ATG GGG GTG CAC GAA TGT CCT GCC TGG CTG TGG CTT           48
                 Met Gly Val His Glu Cys Pro Ala Trp Leu Trp Leu
                     225                 230                 235
   CTC CTG TCC CTG CTG TCG CTC CCT CTG GGC CTC CCA GTC CTG GGC GAC         96
   Leu Leu Ser Leu Leu Ser Leu Pro Leu Gly Leu Pro Val Leu Gly Asp
                   240                 245                 250
   TAC AAG GAC GAT GAC GAT AAG GCG GCC GCC ATT CTG GCC CCG GCC ACC        144
   Tyr Lys Asp Asp Asp Asp Lys Ala Ala Ala Ile Leu Ala Pro Ala Thr
               255                 260                 265
   CAC GCT TGT CGA GCG AAT GGG CAA TAT TTC CTC ACA AAT TGT TGT GCC        192
   His Ala Cys Arg Ala Asn Gly Gln Tyr Phe Leu Thr Asn Cys Cys Ala
           270                 275                 280
   CCG GAG GAC ATC GGG TTC TGC CTG GAG GGT GGA TGC CTG GTG GCC CTG        240
   Pro Glu Asp Ile Gly Phe Cys Leu Glu Gly Gly Cys Leu Val Ala Leu
       285                 290                 295
   GGG TGC ACG ATT TGC ACT GAC CAA TGC TGG CCA CTG TAT CAG GCG GGT        288
   Gly Cys Thr Ile Cys Thr Asp Gln Cys Trp Pro Leu Tyr Gln Ala Gly
   300                 305                 310                 315
   TTG GCT GTG CGG CCT GGC AAG TCC GCG GCC CAA CTG GTG GGG GAG CTG        336
   Leu Ala Val Arg Pro Gly Lys Ser Ala Ala Gln Leu Val Gly Glu Leu
                   320                 325                 330
   GGT AGC CTA TAC GGG CCC CTG TCG GTC TCG GCC TAT GTG GCT GGG ATC        384
   Gly Ser Leu Tyr Gly Pro Leu Ser Val Ser Ala Tyr Val Ala Gly Ile
               335                 340                 345
   CTG GGC CTG GGT GAG GTG TAC TCG GGT GTC CTA ACG GTG GGA GTC GCG        432
   Leu Gly Leu Gly Glu Val Tyr Ser Gly Val Leu Thr Val Gly Val Ala
           350                 355                 360
   TTG ACG CGC CGG ATC TAC CCG GTG CCT AAC CTG ACG TGT GCA GTC GCG        480
   Leu Thr Arg Arg Ile Tyr Pro Val Pro Asn Leu Thr Cys Ala Val Ala
       365                 370                 375
   TGT GAG TTA AAG TGG GAA AGT GAG TTT TGG AGA TGG ACT GAA CAG CTG        528
   Cys Glu Leu Lys Trp Glu Ser Glu Phe Trp Arg Trp Thr Glu Gln Leu
   380                 385                 390                 395
   GCC TCC AAC TAC TGG ATT CTG GAA TAATAGTCTA GA                          564
   Ala Ser Asn Tyr Trp Ile Leu Glu
                   400
(2) ANGABEN ZU SEQ ID NO: 4:
   (i) SEQUENZKENNZEICHEN:
      (A) LÄNGE: 180 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Gly Val His Glu Cys Pro Ala Trp Leu Trp Leu Leu Leu Ser Leu
    1               5                  10                  15
   Leu Ser Leu Pro Leu Gly Leu Pro Val Leu Gly Asp Tyr Lys Asp Asp
               20                  25                  30
   Asp Asp Lys Ala Ala Ala Ile Leu Ala Pro Ala Thr His Ala Cys Arg
               35                  40                  45
   Ala Asn Gly Gln Tyr Phe Leu Thr Asn Cys Cys Ala Pro Glu Asp Ile
           50                  55                  60
   Gly Phe Cys Leu Glu Gly Gly Cys Leu Val Ala Leu Gly Cys Thr Ile
    65                  70                  75                  80
   Cys Thr Asp Gln Cys Trp Pro Leu Tyr Gln Ala Gly Leu Ala Val Arg
                        85                  90                  95
   Pro Gly Lys Ser Ala Ala Gln Leu Val Gly Glu Leu Gly Ser Leu Tyr
               100                 105                 110
   Gly Pro Leu Ser Val Ser Ala Tyr Val Ala Gly Ile Leu Gly Leu Gly
           115                 120                 125
   Glu Val Tyr Ser Gly Val Leu Thr Val Gly Val Ala Leu Thr Arg Arg
       130                 135                 140
   Ile Tyr Pro Val Pro Asn Leu Thr Cys Ala Val Ala Cys Glu Leu Lys
   145                 150                 155                 160
   Trp Glu Ser Glu Phe Trp Arg Trp Thr Glu Gln Leu Ala Ser Asn Tyr
                   165                 170                 175
   Trp Ile Leu Glu
               180
(2) ANGABEN ZU SEQ ID NO: 5:
   (i) SEQUENZKENNZEICHEN:
      (A) LÄNGE: 1302 Basenpaare
      (B) ART: Nucleotid
      (C) STRANGFORM: beides
      (D) TOPOLOGIE: linear
   (vi) URSPRÜNLICHE HERKUNFT:
      (A) ORGANISMUS: Hepatitis G Virus
   (vii) UNMITTELBARE HERKUNFT:
      (B) CLON(E) : E2TM
   (ix) MERKMAL:
      (A) NAME/SCHLÜSSEL: CDS
      (B) LAGE:13..1290
   (xi) SEQUENZBESCHREIBUNG: SEQ ID NO: 5:
   GAATTCACCA CC ATG GGG GTG CAC GAA TGT CCT GCC TGG CTG TGG CTT      48
                 Met Gly Val His Glu Cys Pro Ala Trp Leu Trp Leu
                                         185                 190
   CTC CTG TCC CTG CTG TCG CTC CCT CTG GGC CTC CCA GTC CTG GGC GAC    96
   Leu Leu Ser Leu Leu Ser Leu Pro Leu Gly Leu Pro Val Leu Gly Asp
           195                 200                 205
   TAC AAG GAC GAT GAC GAT AAG GCG GCC GCG ATG TCG CAA GGC GCC CCT   144
   Tyr Lys Asp Asp Asp Asp Lys Ala Ala Ala Met Ser Gln Gly Ala Pro
       210                 215                 220
   GCC TCC GTT TTG GGG TCA CGC CCC TTT GAC TAC GGG TTG ACT TGG CAG   192
   Ala Ser Val Leu Gly Ser Arg Pro Phe Asp Tyr Gly Leu Thr Trp Gln
   225                 230                 235                 240
   ACC TGC TCT TGC AGG GCC AAC GGT TCG CGT TTT TCG ACT GGG GAG AAG   240
   Thr Cys Ser Cys Arg Ala Asn Gly Ser Arg Phe Ser Thr Gly Glu Lys
                   245                 250                 255
   GTG TGG GAC CGT GGG AAC GTT ACG CTT CAG TGT GAC TGC CCT AAC GGC   288
   Val Trp Asp Arg Gly Asn Val Thr Leu Gln Cys Asp Cys Pro Asn Gly
               260                 265                 270
   CCC TGG GTG TGG TTG CCA GCC TTT TGC CAA GCA ATC GGC TGG GGT GAC   336
   Pro Trp Val Trp Leu Pro Ala Phe Cys Gln Ala Ile Gly Trp Gly Asp
           275                 280                 285
   CCC ATC ACT TAT TGG AGC CAC GGG CAA AAT CAG TGG CCC CTT TCA TGC   384
   Pro Ile Thr Tyr Trp Ser His Gly Gln Asn Gln Trp Pro Leu Ser Cys
       290                 295                 300
   CCC CAG TAT GTC TAT GGG TCT GCT ACA GTC ACT TGC GTG TGG GGT TCC        432
   Pro Gln Tyr Val Tyr Gly Ser Ala Thr Val Thr Cys Val Trp Gly Ser
   305                 310                 315                 320
   GCT TCT TGG TAT GCC TCC ACC AGT GGT CGC GAC TCG AAG ATA GAT GTG        480
   Ala Ser Trp Tyr Ala Ser Thr Ser Gly Arg Asp Ser Lys Ile Asp Val
                   325                 330                 335
   TGG AGT TTA GTG CCA GTT GGC TCT GCC ACC TGC ACC ATA GCC GCA CTT        528
   Trp Ser Leu Val Pro Val Gly Ser Ala Thr Cys Thr Ile Ala Ala Leu
               340                 345                 350
   GGA TCA TCG GAT CGC GAC ACG GTG CCT GGG CTC TCC GAG TGG GGA ATC        576
   Gly Ser Ser Asp Arg Asp Thr Val Pro Gly Leu Ser Glu Trp Gly Ile
           355                 360                 365
   CCG TGC GTG ACG TGT GTT CTG GAC CGT CGG CCT GCT TCA TGC GGC ACC        624
   Pro Cys Val Thr Cys Val Leu Asp Arg Arg Pro Ala Ser Cys Gly Thr
       370                 375                 380
   TGT GTG AGG GAC TGC TGG CCC GAG ACC GGG TCG GTT AGG TTC CCA TTC        672
   Cys Val Arg Asp Cys Trp Pro Glu Thr Gly Ser Val Arg Phe Pro Phe
   385                 390                 395                 400
   CAT CGG TGC GGC GTG GGG CCT CGG CTG ACA AAG GAC TTG GAA GCT GTG        720
   His Arg Cys Gly Val Gly Pro Arg Leu Thr Lys Asp Leu Glu Ala Val
                   405                 410                 415
   CCC TTC GTC AAT AGG ACA ACT CCC TTC ACC ATT AGG GGG CCC CTG GGC        768
   Pro Phe Val Asn Arg Thr Thr Pro Phe Thr Ile Arg Gly Pro Leu Gly
               420                 425                 430
   AAC CAG GGC CGA GGC AAC CCG GTG CGG TCG CCC TTG GGT TTT GGG TCC        816
   Asn Gln Gly Arg Gly Asn Pro Val Arg Ser Pro Leu Gly Phe Gly Ser
           435                 440                 445
   TAC GCC ATG ACC AGG ATC CGA GAT ACC CTA CAT CTG GTG GAG TGT CCC        864
   Tyr Ala Met Thr Arg Ile Arg Asp Thr Leu His Leu Val Glu Cys Pro
       450                 455                 460
   ACA CCA GCC ATC GAG CCT CCC ACC GGG ACG TTT GGG TTC TTC CCC GGG        912
   Thr Pro Ala Ile Glu Pro Pro Thr Gly Thr Phe Gly Phe Phe Pro Gly
   465                 470                 475                 480
   ACG CCG CCT CTC AAC AAC TGC ATG CTC TTG GGC ACG GAA GTG TCC GAG        960
   Thr Pro Pro Leu Asn Asn Cys Met Leu Leu Gly Thr Glu Val Ser Glu
                   485                 490                 495
   GCA CTT GGG GGG GCT GGC CTC ACG GGG GGG TTC TAT GAA CCC CTG GTG       1008
   Ala Leu Gly Gly Ala Gly Leu Thr Gly Gly Phe Tyr Glu Pro Leu Val
               500                 505                 510
   CGC AGG TGT TCG GAG CTG ATG GGA AGC CGA AAT CCG GTT TGT CCG GGG       1056
   Arg Arg Cys Ser Glu Leu Met Gly Ser Arg Asn Pro Val Cys Pro Gly
           515                 520                 525
   TTT GCA TGG CTC TCT TCG GGC AGG CCT GAT GGG TTT ATA CAT GTC CAG       1104
   Phe Ala Trp Leu Ser Ser Gly Arg Pro Asp Gly Phe Ile His Val Gln
       530                 535                 540
   GGT CAC TTG CAG GAG GTG GAT GCA GGC AAC TTC ATC CCG CCC CCG CGC       1152
   Gly His Leu Gln Glu Val Asp Ala Gly Asn Phe Ile Pro Pro Pro Arg
   545                 550                 555                 560
   TGG TTG CTC TTG GAC TTT GTA TTT GTC CTG TTA TAC CTG ATG AAG CTG       1200
   Trp Leu Leu Leu Asp Phe Val Phe Val Leu Leu Tyr Leu Met Lys Leu
                   565                 570                 575
   GCT GAG GCA CGG TTG GTC CCG CTG ATC TTG CTG CTG CTA TGG TGG TGG       1248
   Ala Glu Ala Arg Leu Val Pro Leu Ile Leu Leu Leu Leu Trp Trp Trp
               580                 585                 590
   GTG AAC CAG CTG GCA GTC CTA GGG CTG CCG GCT GTG GAA GCC               1290
   Val Asn Gln Leu Ala Val Leu Gly Leu Pro Ala Val Glu Ala
           595                 600                 605
   TAATAGTCTA GA                                                         1302
(2) ANGABEN ZU SEQ ID NO: 6:
   (i) SEQUENZKENNZEICHEN:
      (A) LÄNGE: 426 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Gly Val His Glu Cys Pro Ala Trp Leu Trp Leu Leu Leu Ser Leu
     1               5                  10                  15
   Leu Ser Leu Pro Leu Gly Leu Pro Val Leu Gly Asp Tyr Lys Asp Asp
                20                  25                  30
   Asp Asp Lys Ala Ala Ala Met Ser Gln Gly Ala Pro Ala Ser Val Leu
            35                  40                  45
   Gly Ser Arg Pro Phe Asp Tyr Gly Leu Thr Trp Gln Thr Cys Ser Cys
        50                  55                  60
   Arg Ala Asn Gly Ser Arg Phe Ser Thr Gly Glu Lys Val Trp Asp Arg
    65                  70                  75                  80
   Gly Asn Val Thr Leu Gln Cys Asp Cys Pro Asn Gly Pro Trp Val Trp
                    85                  90                  95
   Leu Pro Ala Phe Cys Gln Ala Ile Gly Trp Gly Asp Pro Ile Thr Tyr
               100                 105                 110
   Trp Ser His Gly Gln Asn Gln Trp Pro Leu Ser Cys Pro Gln Tyr Val
           115                 120                 125
   Tyr Gly Ser Ala Thr Val Thr Cys Val Trp Gly Ser Ala Ser Trp Tyr
       130                 135                 140
   Ala Ser Thr Ser Gly Arg Asp Ser Lys Ile Asp Val Trp Ser Leu Val
   145                 150                 155                 160
   Pro Val Gly Ser Ala Thr Cys Thr Ile Ala Ala Leu Gly Ser Ser Asp
                   165                 170                 175
   Arg Asp Thr Val Pro Gly Leu Ser Glu Trp Gly Ile Pro Cys Val Thr
               180                 185                 190
   Cys Val Leu Asp Arg Arg Pro Ala Ser Cys Gly Thr Cys Val Arg Asp
           195                 200                 205
   Cys Trp Pro Glu Thr Gly Ser Val Arg Phe Pro Phe His Arg Cys Gly
       210                 215                 220
   Val Gly Pro Arg Leu Thr Lys Asp Leu Glu Ala Val Pro Phe Val Asn
   225                 230                 235                 240
   Arg Thr Thr Pro Phe Thr Ile Arg Gly Pro Leu Gly Asn Gln Gly Arg
                   245                 250                 255
   Gly Asn Pro Val Arg Ser Pro Leu Gly Phe Gly Ser Tyr Ala Met Thr
               260                 265                 270
   Arg Ile Arg Asp Thr Leu His Leu Val Glu Cys Pro Thr Pro Ala Ile
           275                 280                 285
   Glu Pro Pro Thr Gly Thr Phe Gly Phe Phe Pro Gly Thr Pro Pro Leu
       290                 295                 300
   Asn Asn Cys Met Leu Leu Gly Thr Glu Val Ser Glu Ala Leu Gly Gly
   305                 310                 315                 320
   Ala Gly Leu Thr Gly Gly Phe Tyr Glu Pro Leu Val Arg Arg Cys Ser
                   325                 330                 335
   Glu Leu Met Gly Ser Arg Asn Pro Val Cys Pro Gly Phe Ala Trp Leu
               340                 345                 350
   Ser Ser Gly Arg Pro Asp Gly Phe Ile His Val Gln Gly His Leu Gln
           355                 360                 365
   Glu Val Asp Ala Gly Asn Phe Ile Pro Pro Pro Arg Trp Leu Leu Leu
       370                 375                 380
   Asp Phe Val Phe Val Leu Leu Tyr Leu Met Lys Leu Ala Glu Ala Arg
   385                 390                 395                 400
   Leu Val Pro Leu Ile Leu Leu Leu Leu Trp Trp Trp Val Asn Gln Leu
                   405                 410                 415
   Ala Val Leu Gly Leu Pro Ala Val Glu Ala
               420                 425
(2) ANGABEN ZU SEQ ID NO: 7:
   (i) SEQUENZKENNZEICHEN:
      (A) LÄNGE: 1146 Basenpaare
      (B) ART: Nucleotid
      (C) STRANGFORM: beides
      (D) TOPOLOGIE: linear
   (vi) URSPRÜNLICHE HERKUNFT:
      (A) ORGANISMUS: Hepatitis G Virus
   (vii) UNMITTELBARE HERKUNFT:
      (B) CLON(E) : E2S
   (ix) MERKMAL:
      (A) NAME/SCHLÜSSEL: CDS
      (B) LAGE:13..1134
   (xi) SEQUENZBESCHREIBUNG: SEQ ID NO: 7:
   GAATTCACCA CC ATG GGG GTG CAC GAA TGT CCT GCC TGG CTG TGG CTT          48
                 Met Gly Val His Glu Cys Pro Ala Trp Leu Trp Leu
                             430                 435
   CTC CTG TCC CTG CTG TCG CTC CCT CTG GGC CTC CCA GTC CTG GGC GAC        96
   Leu Leu Ser Leu Leu Ser Leu Pro Leu Gly Leu Pro Val Leu Gly Asp
       440                 445                 450
   TAC AAG GAC GAT GAC GAT AAG GCG GCC GCG ATG TCG CAA GGC GCC CCT       144
   Tyr Lys Asp Asp Asp Asp Lys Ala Ala Ala Met Ser Gln Gly Ala Pro
   455                 460                 465                 470
   GCC TCC GTT TTG GGG TCA CGC CCC TTT GAC TAC GGG TTG ACT TGG CAG       192
   Ala Ser Val Leu Gly Ser Arg Pro Phe Asp Tyr Gly Leu Thr Trp Gln
                   475                 480                 485
   ACC TGC TCT TGC AGG GCC AAC GGT TCG CGT TTT TCG ACT GGG GAG AAG       240
   Thr Cys Ser Cys Arg Ala Asn Gly Ser Arg Phe Ser Thr Gly Glu Lys
               490                 495                 500
   GTG TGG GAC CGT GGG AAC GTT ACG CTT CAG TGT GAC TGC CCT AAC GGC       288
   Val Trp Asp Arg Gly Asn Val Thr Leu Gln Cys Asp Cys Pro Asn Gly
           505                 510                 515
   CCC TGG GTG TGG TTG CCA GCC TTT TGC CAA GCA ATC GGC TGG GGT GAC       336
   Pro Trp Val Trp Leu Pro Ala Phe Cys Gln Ala Ile Gly Trp Gly Asp
       520                 525                 530
   CCC ATC ACT TAT TGG AGC CAC GGG CAA AAT CAG TGG CCC CTT TCA TGC         384
   Pro Ile Thr Tyr Trp Ser His Gly Gln Asn Gln Trp Pro Leu Ser Cys
   535                 540                 545                 550
   CCC CAG TAT GTC TAT GGG TCT GCT ACA GTC ACT TGC GTG TGG GGT TCC         432
   Pro Gln Tyr Val Tyr Gly Ser Ala Thr Val Thr Cys Val Trp Gly Ser
                   555                 560                 565
   GCT TCT TGG TAT GCC TCC ACC AGT GGT CGC GAC TCG AAG ATA GAT GTG         480
   Ala Ser Trp Tyr Ala Ser Thr Ser Gly Arg Asp Ser Lys Ile Asp Val
               570                 575                 580
   TGG AGT TTA GTG CCA GTT GGC TCT GCC ACC TGC ACC ATA GCC GCA CTT         528
   Trp Ser Leu Val Pro Val Gly Ser Ala Thr Cys Thr Ile Ala Ala Leu
           585                 590                 595
   GGA TCA TCG GAT CGC GAC ACG GTG CCT GGG CTC TCC GAG TGG GGA ATC         576
   Gly Ser Ser Asp Arg Asp Thr Val Pro Gly Leu Ser Glu Trp Gly Ile
       600                 605                 610
   CCG TGC GTG ACG TGT GTT CTG GAC CGT CGG CCT GCT TCA TGC GGC ACC         624
   Pro Cys Val Thr Cys Val Leu Asp Arg Arg Pro Ala Ser Cys Gly Thr
   615                 620                 625                 630
   TGT GTG AGG GAC TGC TGG CCC GAG ACC GGG TCG GTT AGG TTC CCA TTC         672
   Cys Val Arg Asp Cys Trp Pro Glu Thr Gly Ser Val Arg Phe Pro Phe
                   635                 640                 645
   CAT CGG TGC GGC GTG GGG CCT CGG CTG ACA AAG GAC TTG GAA GCT GTG         720
   His Arg Cys Gly Val Gly Pro Arg Leu Thr Lys Asp Leu Glu Ala Val
               650                 655                 660
   CCC TTC GTC AAT AGG ACA ACT CCC TTC ACC ATT AGG GGG CCC CTG GGC         768
   Pro Phe Val Asn Arg Thr Thr Pro Phe Thr Ile Arg Gly Pro Leu Gly
           665                 670                 675
   AAC CAG GGC CGA GGC AAC CCG GTG CGG TCG CCC TTG GGT TTT GGG TCC         816
   Asn Gln Gly Arg Gly Asn Pro Val Arg Ser Pro Leu Gly Phe Gly Ser
       680                 685                 690
   TAC GCC ATG ACC AGG ATC CGA GAT ACC CTA CAT CTG GTG GAG TGT CCC         864
   Tyr Ala Met Thr Arg Ile Arg Asp Thr Leu His Leu Val Glu Cys Pro
   695                 700                 705                 710
   ACA CCA GCC ATC GAG CCT CCC ACC GGG ACG TTT GGG TTC TTC CCC GGG        912
   Thr Pro Ala Ile Glu Pro Pro Thr Gly Thr Phe Gly Phe Phe Pro Gly
                   715                 720                 725
   ACG CCG CCT CTC AAC AAC TGC ATG CTC TTG GGC ACG GAA GTG TCC GAG        960
   Thr Pro Pro Leu Asn Asn Cys Met Leu Leu Gly Thr Glu Val Ser Glu
               730                 735                 740
   GCA CTT GGG GGG GCT GGC CTC ACG GGG GGG TTC TAT GAA CCC CTG GTG       1008
   Ala Leu Gly Gly Ala Gly Leu Thr Gly Gly Phe Tyr Glu Pro Leu Val
           745                 750                 755
   CGC AGG TGT TCG GAG CTG ATG GGA AGC CGA AAT CCG GTT TGT CCG GGG       1056
   Arg Arg Cys Ser Glu Leu Met Gly Ser Arg Asn Pro Val Cys Pro Gly
       760                 765                 770
   TTT GCA TGG CTC TCT TCG GGC AGG CCT GAT GGG TTT ATA CAT GTC CAG       1104
   Phe Ala Trp Leu Ser Ser Gly Arg Pro Asp Gly Phe Ile His Val Gln
   775                 780                 785                 790
   GGT CAC TTG CAG GAG GTG GAT GCA GGC AAC TAATAGTCTA GA                 1146
   Gly His Leu Gln Glu Val Asp Ala Gly Asn
                   795                 800
(2) ANGABEN ZU SEQ ID NO: 8:
   (i) SEQUENZKENNZEICHEN:
      (A) LÄNGE: 374 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8:
   Met Gly Val His Glu Cys Pro Ala Trp Leu Trp Leu Leu Leu Ser Leu
     1               5                  10                  15
   Leu Ser Leu Pro Leu Gly Leu Pro Val Leu Gly Asp Tyr Lys Asp Asp
               20                  25                  30
   Asp Asp Lys Ala Ala Ala Met Ser Gln Gly Ala Pro Ala Ser Val Leu
   35 40 45
   Gly Ser Arg Pro Phe Asp Tyr Gly Leu Thr Trp Gln Thr Cys Ser Cys
   50 55 60
   Arg Ala Asn Gly Ser Arg Phe Ser Thr Gly Glu Lys Val Trp Asp Arg
   65 70 75 80
   Gly Asn Val Thr Leu Gln Cys Asp Cys Pro Asn Gly Pro Trp Val Trp
   85 90 95
   Leu Pro Ala Phe Cys Gln Ala Ile Gly Trp Gly Asp Pro Ile Thr Tyr
   100 105 110
   Trp Ser His Gly Gln Asn Gln Trp Pro Leu Ser Cys Pro Gln Tyr Val
   115 120 125
   Tyr Gly Ser Ala Thr Val Thr Cys Val Trp Gly Ser Ala Ser Trp Tyr
   130 135 140
   Ala Ser Thr Ser Gly Arg Asp Ser Lys Ile Asp Val Trp Ser Leu Val
   145 150 155 160
   Pro Val Gly Ser Ala Thr Cys Thr Ile Ala Ala Leu Gly Ser Ser Asp
   165 170 175
   Arg Asp Thr Val Pro Gly Leu Ser Glu Trp Gly Ile Pro Cys Val Thr
   180 185 190
   Cys Val Leu Asp Arg Arg Pro Ala Ser Cys Gly Thr Cys Val Arg Asp
   195 200 205
   Cys Trp Pro Glu Thr Gly Ser Val Arg Phe Pro Phe His Arg Cys Gly
   210 215 220
   Val Gly Pro Arg Leu Thr Lys Asp Leu Glu Ala Val Pro Phe Val Asn
   225 230 235 240
   Arg Thr Thr Pro Phe Thr Ile Arg Gly Pro Leu Gly Asn Gln Gly Arg
   245 250 255
   Gly Asn Pro Val Arg Ser Pro Leu Gly Phe Gly Ser Tyr Ala Met Thr
   260 265 270
   Arg Ile Arg Asp Thr Leu His Leu Val Glu Cys Pro Thr Pro Ala Ile
           275                 280                 285
   Glu Pro Pro Thr Gly Thr Phe Gly Phe Phe Pro Gly Thr Pro Pro Leu
       290                 295                 300
   Asn Asn Cys Met Leu Leu Gly Thr Glu Val Ser Glu Ala Leu Gly Gly
   305                 310                 315                 320
   Ala Gly Leu Thr Gly Gly Phe Tyr Glu Pro Leu Val Arg Arg Cys Ser
                   325                 330                 335
   Glu Leu Met Gly Ser Arg Asn Pro Val Cys Pro Gly Phe Ala Trp Leu
               340                 345                 350
   Ser Ser Gly Arg Pro Asp Gly Phe Ile His Val Gln Gly His Leu Gln
           355                 360                 365
   Glu Val Asp Ala Gly Asn
       370